# EUROPEAN PATENT SPECIFICATION

(11) **EP 2 167 036 B1**
(45) Date of publication and mention of the grant of the patent: **18.09.2013**
(21) Application number: 08794464.1
(22) Date of filing: 11.07.2008
(51) Int. Cl.: A61K 9/00, A61K 47/20, A61K 47/24

(54) **COMPOSITIONS FOR DELIVERING A DRUG**
ZUSAMMENSETZUNGEN ZUR VERABREICHUNG EINES ARZNEIMITTELS
COMPOSITIONS POUR ADMINISTRER UN MÉDICAMENT

(30) Priority: 11.07.2007 US 949042 P
(43) Date of publication of application: 31.03.2010
(73) Proprietor: Dow Corning Corporation, Midland, Michigan 48686-0994 (US)
(72) Inventor: RAUL, Victor, Albert, Midland, MI 48642 (US); NARTKER, Linda, Sue, Midland, MI 48642 (US); HUBER, Robert, O., Midland, MI 48642 (US)
(74) Representative: Neilson, Martin Mark
(86) International application number: PCT/US2008/008544
(87) International publication number: WO 2009/009134

(56) References cited:
- WO-A-97/48387
- WO-A-2005/123092
- WO-A-2006/131401
- WO-A-2006/134272

## Description

### CROSS-REFERENCE TO RELATED APPLICATIONS

This application claims the benefit of U.S. Provisional Patent Application Serial No. 60/949,042, which was filed on July 11, 2007.

### FIELD OF THE INVENTION

The present invention relates to a composition for delivering a drug, and more specifically, to a composition comprising a mixture of a silicone and a solvent, a drug, a salt of a dialkyl sulfosuccinate, and a solubilizer.

### DESCRIPTION OF THE RELATED ART

Compositions that include drugs, such as retinoids, are well known in the healthcare art. These compositions are typically emulsions, i.e., include water, and include petrolatum or polyethylene components, which are tactilely greasy. When a composition is greasy, patients are less likely to use or reuse the composition, thereby decreasing patient compliance. Patient compliance is further compromised when crystals, polymorphs, and agglomerations of the crystals, of the drug, form over time. Uncontrolled crystal formation and agglomeration in the compositions yields crystals having an average diameter of greater than 10 microns, typically ranging from 15 to 20 microns or higher. Such uncontrolled crystal formation and agglomerations thereof result in physical instability of the compositions, decrease in bioavailability and/or bioequivalence.

The aforementioned compositons are generally used to treat skin conditions, such as acne or psoriais. For example, the aforementioned compositions can be used for soothing affected skin and reducing dryness which accompanies the build-up of psoriatic scales or plaques. Typically, such compositions are in the form of topical compositions, and these topical compositions, when used to treat psorirasis, are applied directly to the psoriatic scales or plaques. Such topical application can help reduce inflammation, remove built-up scale, reduce skin turn over, and clear affected skin of plaques. Unfortunately, these topical compositions often irritate normal skin surrounding affected areas, can be time consuming and awkward to apply, cannot be used for long periods of time, and can stain clothing or have a strong odor. As a result, it is sometimes difficult for people to maintain the regular application of these medications. Abrupt withdrawal of applying some topical compositions by a patient, can cause an aggressive recurrence of the skin condition. When topical compositions are used to treat facial acne, retionoids often cause an initial flare up of acne and facial flushing.
[0004a] Additionally, WO 2006/134272; WO 2006/131401; WO 2005/123092; and WO 97/48387 disclose pharmaceutical compositions and methods of preparing the same for topical treatment of a range of disorders.

Accordingly, there remains an opportunity to provide improved compositions that provide desirable aesthetic properties, such as non-greasiness after application to skin, and no or minimal irritation, all of which are key to patient compliance.

### SUMMARY OF THE INVENTION AND ADVANTAGES

The present invention provides a composition comprising a mixture of a silicone and a solvent, a drug, a salt of a dialkyl sulfosuccinate, and a solubilizer. The salt of a dialkyl sulfosuccinate solubilizes the drug. The solubilizer solubilizes the salt of a dialkyl sulfosuccinate. The present invention further provides a method of delivering a drug to a substrate, and a method of forming the composition. According to the present invention, the drug is selected from the group of retinoids, retinyls, vitamin A esters, vitamin D analogues, and combinations thereof.

The present invention provides a unique combination of the mixture of the silicone and the solvent, the drug, the salt of a dialkyl sulfosuccinate, and the solubilizer to make the composition. Drugs of the above group with a wide solubility range are soluble and compatible in the compositions of the present invention. Amounts of the salt of a dialkyl sulfosuccinate and the solubilizer can be adjusted to maintain near saturation of the drug for optimum delivery and release of the drug into substrates such as skin, and therapeutic effectiveness of the drug does not change over time. The composition does not have a deleterious medium that can cause instability of the drug. In addition, water, pH adjustment, and heat are generally not required for preparing the compositions. The compositions can also have desirable aesthetic properties, specifically, tactile feel, such as non-greasiness after application to skin, and can be less irritating.

### DETAILED DESCRIPTION OF THE INVENTION

The composition of this invention may be used for various applications known to those skilled in the healthcare art, typically for topical degrees of drug penetration on and into skin. Examples of skin penetration include, but are not limited to, stratum corneum, viable epidermis, dermis, and systemic (transdermal). The composition of the present invention is especially suited for use in semisolid form as a topical composition, e.g. as a liquid lotion, for use on skin, wherein one or more of the components, such as a drug, can permeate into one or more layers of the skin. As such, the composition can be used for treating various ailments, and is especially useful for treatment of skin disorders, such as psoriasis, and for treatment of follicular diseases, such as acne. The composition can also be used in semi-solid or solid forms such as for transdermal patches and implantable devices.

The composition is typically anhydrous. By "anhydrous", it is meant that the composition is substantially free of water. However, it is to be appreciated that the composition may have some water content due to residual water in one or more of the components of the composition, and/or due to atmospheric moisture absorbed by the composition. If water is present in the composition, the composition typically includes water in an amount less than 5, alternatively less than 1, alternatively approaching or equaling 0, parts by weight, based on 100 parts by weight of the composition. Water is normally undesirable in the composition because water can cause the composition to become unstable and separate over time depending in part on the specific components, and the amounts thereof, employed to make the composition. Water can also cause instability or degradation of certain components, such as a drug, which is described further below, and may cause uncontrolled side reactions of the components employed to make the composition. As such, the composition of the present invention is not an emulsion, which would include water as a primary component. For example, emulsions typically include water in an amount greater than 25, alternatively greater than 50, parts by weight, based on 100 parts by weight of the emulsion.

Generally, the composition is substantially free of an alcohol having the formula CₙH₂ₙ₊₁OH wherein n is from 1 to 5, alternatively from 1 to 2. Such alcohols are considered "lower alcohols" by those skilled in the art, and can also be referred to as monomeric alcohols, which are different than larger compounds that happen to have one or more hydroxyl groups. Examples of lower alcohols include methanol, ethanol, propanol, butanol, pentanol, hexanol, heptanol, octanol, nonanol, decanol, and combinations thereof. It is to be appreciated that the alcohol may also be various isomers of the lower alcohols described above, such as 1-propanol, 1-butanol, 1-pentanol, etc. By "substantially free", it is meant that the composition typically includes the alcohol in an amount less than 5, alternatively less than 1, alternatively approaching or equaling 0, parts by weight, based on 100 parts by weight of the composition. The alcohol is normally undesirable in the composition because the alcohol can act as an irritant when the composition is employed by a consumer. Excessive drying is a problem when compositions employing higher amounts of alcohol are applied on the skin of a consumer. However, in certain embodiments, low amounts of the alcohol, such as those amounts described above, can increase drug solubility, or permeation of the drug (described further below).

The composition comprises a mixture of a silicone and a solvent, a drug, a salt of a dialkyl sulfosuccinate, and a solubilizer. In one embodiment, the silicone comprises a silicone elastomer. In another embodiment, the silicone comprises a silicone adhesive, e.g. a silicone pressure sensitive adhesive (PSA). Specific embodiments are described in further detail below.

Silicone elastomers useful herein typically comprise the reaction product of an ≡Si-H containing polysiloxane and an alpha, omega-diene. The ≡Si-H containing polysiloxane can have the formula

R₃SiO(R'₂SiO)ₐ(R"HSiO)_{b}SiR₃ (I)

wherein R, R', and R" are typically each independently alkyl groups with 1 to 6 carbon atoms, a is 0 to 250, and b is 1 to 250. Other suitable ≡Si-H containing polysiloxanes for making the silicone elastomer include those having the formula

HR₂SiO(R'₂SiO)_{c}SiR₂H (II)

and/or the formula

HR₂SiO(R'₂SiO)ₐ(R"HSiO)ₕSiR₂H (III)

wherein R, R', and R" are typically each independently alkyl groups with 1 to 6 carbon atoms, a is 0 to 250, b is 1 to 250, and c is 0 to 250. Typically, the ≡Si-H containing polysiloxane comprises polysiloxane (I) and at least one of polysiloxane (II) or (III). The alpha, omega-diene is typically of the formula

CH₂=CH(CH₂)ₓCH=CH₂

wherein x is typically 1 to 20. Suitable alpha, omega-dienes for making the silicone elastomer include, but are not limited to, 1,4-pentadiene, 1,5-hexadiene, 1,6-heptadiene, 1,7-octadiene, 1,8-nonadiene, 1,9-decadiene, 1,11-dodecadiene, 1,13-tetradecadiene; and 1,19-eicosadiene, and combinations thereof.

The silicone elastomer is typically made in the presence of a metal catalyst for reacting and cross-linking the ≡Si-H containing polysiloxane and the alpha, omega-diene. Suitable metal catalysts for making the silicone elastomer include Group VIII transition metal catalysts, such as a platinum catalyst. Other suitable metal catalysts for making the silicone elastomer include, but are not limited to, tin catalysts.

When the silicone is a silicone elastomer, the solvent typically comprises a polysiloxane. The polysiloxane is useful for swelling the silicone elastomer. Specifically, when the solvent is combined with the silicone elastomer to form the mixture, the solvent serves to suspend and swell the silicone elastomer to provide an elastic three-dimensional network or matrix, e.g. a gel, which can quickly be formed by applying shear force to the mixture, such as by using a dispersion blade, a dental mixer, etc., to blend the silicone elastomer and the solvent to form the mixture.

In the mixture of the silicone elastomer and the polysiloxane, the polysiloxane is typically a low molecular weight linear polysiloxane, a low molecular weight cyclic polysiloxane, or a mixture of low molecular weight linear and cyclic polysiloxanes. Suitable polysiloxanes for making the composition are typically selected from the group consisting of, but are not limited to, hexamethyldisiloxane, hexamethylcyclotrisiloxane, hexadecamethylheptasiloxane, octamethyltrisiloxane, octamethylcyclotetrasiloxane, decamethyltetrasiloxane, decamethylcyclopentasiloxane, dodecamethylpentasiloxane, dodecamethylcyclohexasiloxane, tetradecamethylhexasiloxane, and combinations thereof. Typically, the polysiloxane is a cyclosiloxane, more typically decamethylcyclopentasiloxane, which is commonly referred to in the art as "D5". Other suitable polysiloxanes for making the mixture of the silicone elastomer and the solvent include low molecular weight linear alkyl polysiloxanes, low molecular weight aryl polysiloxanes, or mixtures thereof. If employed, the low molecular weight linear polysiloxanes are typically of the formula

R₃SiO(R₂SiO)_{y}SiR₃

wherein R is typically selected from the group of alkyl groups containing 1 to 6 carbon atoms and/or aryl groups such as phenyl, and y is typically a value to impart the solvent with a viscosity generally less than 100 mm²/s.

The solvent, e.g. polysiloxane, is typically a liquid under ambient conditions and typically has a low viscosity for improved application and spreading of the composition onto skin. The solvent, e.g. D5, is typically volatile, to at least partially evaporate after the composition is applied to skin; however, the solvent can be non-volatile, depending on the specific solvent employed to make the composition. The term "volatile", as used herein, means that the solvent has a vapor pressure of more than 0.2 mm Hg at 25°C at one atmosphere and/or has a boiling point of less than 300°C at one atmosphere. It is to be appreciated that the solvent can be present during manufacture of the silicone elastomer. In other words, the solvent can be inherent in the mixture with the silicone elastomer, and/or the solvent can be added after the silicone elastomer is made.

The mixture may be made with the ≡Si-H containing polysiloxane and the alpha, omega-diene to form a gel by crosslinking and addition of SiH across double bonds in the alpha, omega-diene, typically with a nonvolatile content of 8% to 18%, alternatively from 12% to 13%, in a cyclomethicone, e.g. D5. In this embodiment, the mixture is generally assigned the INCI name: "cyclomethicone (and) dimethicone crosspolymer". In another embodiment, the mixture comprises a "dimethicone (and) dimethicone crosspolymer". If employed, the crosspolymer is typically a loosely cross-linked crosspolymer. It is believed that the loose cross-linking of the crosspolymer is useful for imparting the composition with desirable viscosity and aesthetic properties, as further described below.

Specific suitable examples of the mixture of the silicone elastomer and the solvent are Dow Corning^{®} ST-Elastomer 10, Dow Corning^{®} 9040 Silicone Elastomer Blend, and Dow Corning^{®} 9041 Silicone Elastomer Blend, all of which are commercially available from Dow Coming Corporation of Midland, MI. Further suitable mixtures of the silicone elastomer and the solvent are disclosed in U.S. Patent Application Nos. 5,654,362 and 5,888,210, both to Schultz et al., which are incorporated herein by reference in their entirety. The silicone elastomer is typically present in an amount of 5 to 20, alternatively from 10 to 15, alternatively from 12 to 13, parts by weight, based on 100 parts by weight of the mixture. If the solvent is the polysiloxane, the solvent is typically present in an amount of 45 to 90, alternatively from 55 to 90, alternatively from 60 to 88, parts by weight, based on 100 parts by weight of the mixture.

The silicone elastomer and the solvent are typically present in a weight ratio of 5:1 to 9:1. This mixture has a viscosity that is useful for imparting the composition with ease of application. The mixture is typically present in an amount of 70 to 98 parts by weight, based on 100 parts by weight of the composition. However, for purposes of the present invention, it is to be appreciated that various amounts of the mixtures may be used, other than those amounts described herein, such as quantities sufficient (qs) to achieve a desirable property such as viscosity and/or dosing of the drug.

The silicone may also be a silicone adhesive, typically a silicone based pressure sensitive adhesive (PSA), which can be used for transdermal reservoir patches or devices. Typically, the silicone adhesive comprises the reaction product of a polydialkyldimethylsiloxane end blocked with silanols and a silicate resin. In certain embodiments, the silicone adhesive comprises the condensation reaction product of a polydimethylsiloxane end blocked with silanols and a silicate resin. Specific examples of suitable silicone adhesives are Dow Corning^{®} 7-9800 Soft Skin Adhesive, Dow Corning^{®} BIO-PSA 7-4502 Silicone Adhesive, Dow Corning^{®} BIO-PSA 7-4602 Silicone Adhesive, Dow Corning^{®} 7-4101, Dow Corning^{®} 7-4102, Dow Corning^{®} 7-4201, Dow Corning^{®} 7-4202, Dow Corning^{®} 7-4301, Dow Corning^{®} 7-4302, Dow Corning^{®} 7-4401, Dow Corning^{®} 7-402, Dow Corning^{®} 7-4501, Dow Corning^{®} 7-4601, and Dow Corning^{®} 7-4560, all of which are commercially available from Dow Coming Corporation.

When the silicone is the silicone adhesive, the solvent is typically selected from the group of alkanes, such as heptane; arenes, such as toluene; and esters, such as ethyl acetate; and combinations thereof. In this embodiment, the solvent serves as a processing aid, which is further described below. It is to be appreciated that the silicone adhesive may also be applied to other substrates known in the art, such as patches. The silicone adhesive is typically present in at least 50 parts by weight based on 100 parts of the silicone adhesive and the solvent combined, i.e., the mixture. If employed, the silicone adhesive is typically present in an amount of 40 to 80, alternatively from 50 to 70, alternatively from 60 to 65, parts by weight, based on 100 parts by weight of the mixture. The solvent, e.g. ethyl acetate, is typically present in an amount of 1 to 40, alternatively from 25 to 40, alternatively from 25 to 30, parts by weight, based on 100 parts by weight of the mixture.

The silicone may also be a dimethiconol, which is a dimethyl siloxane terminated with hydroxyl groups. Specific examples of suitable dimethiconols are Dow Corning^{®} Dimethiconal Blend 20 and ST-Dimethiconol 40, both of which are commercially available from Dow Coming Corporation. Other suitable solvents, for purposes of the present invention, include Dow Corning^{®} 200 Fluid (e.g. Dow Corning^{®} 200 Fluid, 5 CST), Dow Corning^{®} 245 Fluid, dodecane, isododecane, isohexadecane, and isodecylneopentanoate.

Some of the mixtures described above and other mixtures of the silicone and the solvent suitable for making the composition are disclosed by various patents and publications including U.S. Patent Application Nos. 4,882,377 to Sweet et al., 4,987,169 to Kuwata et al., 5,599,533 to Stepniewski et al., 5,654,362 to Schulz Jr. et al., 5,811,487 to Schulz Jr. et al., 5,880,210 to Schulz Jr. et al., 5,889,108 to Zhang, 5,929,164 to Zhang, 5,948, 855 to Lin et al, 5,969,035 to Meinhardt et al., 5,977,280 to Kadlec et al., 5,994,459 to Berg et al., 6,015,858 to Gornowicz, 6,027,738 to Stepniewski et al., 6,080,394 to Lin et al., 6,168,782 to Lin et al., 6,177,071 to Lin et al., 6,200,581 to Lin et al., 6,207,717 to Lin et al., 6,221,927 to Lin et al., 6,221,979 to Lin et al., 6,238,657 to Lin et al., 6,346,583 to Kilgour et al., 6,444,745 to Kilgour et al., 6,538,061 to Chaiyawat et al., and U.S. Patent Application Publication No. 2004/0228821 to Sunkel et al. It is to be appreciated that the mixture of the present invention may comprise any combination of one or more of the silicones and one or more of the solvents described and exemplified above.

The mixture of the silicone elastomer and the solvent is typically in the form of the gel, which imparts the composition with desirable viscosity and aesthetic properties, improves stability of the composition, and suspends and carries the drug within the composition, which is further described below. When the composition has desirable aesthetic properties, specifically, tactile feel, it is believed that consumers are more likely to use or comply with use of the composition due to increased sensory satisfaction and ease of application. This increases overall patient compliance. For example, after the composition is topically applied to the consumer's skin and at least partially absorbed and/or evaporated, some representative aesthetic properties of the post-applied composition include film residue, greasiness, silkiness, tackiness, slipperiness, and gloss. If the composition is compared to another composition known in the art, such as petrolatum, e.g. petroleum jelly, the composition of the present invention generally has lower film residue, lower greasiness, lower tackiness, and lower gloss, and generally has higher silkiness and higher slipperiness, relative to the petrolatum. These aesthetic properties of the composition are typically more desired by the patient relative to the properties of the petrolatum. While the gel is generally employed, the mixture of the silicone elastomer and the solvent can also be in other forms known to those of ordinary skill in the healthcare art. For example, and as alluded to above, the mixture can be a liquid, a semi-solid, a blend, or a paste, which imparts the composition with the corresponding viscosity and aesthetic properties of the mixture.

The drug is generally a drug suitable for treatment of acne or psoriasis. The retinoid and psoriasis drugs are lipophilic. Such drugs are well known to those skilled in the healthcare art. The drug is selected from the group of retinoids, retinyls, vitamin A esters, vitamin D analogues, and combinations thereof. Examples of suitable retinoids, which are also classified for purposes of the present invention as drugs that are pharmacologically effective via binding with retinoid receptors in the skin include, but are not limited to, retinoic acid, cis-retinoic acid/isotretinoin, trans-retinoic acid/tretinoin, tazarotene, and adapalene. It is to be appreciated that suitable drugs, for purposes of the present invention, generally include all natural and/or synthetic analogues of Vitamin A or retinol-like compounds which possess the biological activity of Vitamin A in the skin as well as the stereoisomers, such as cis-trans isomerism, or geometric isomers of these compounds, such as all-trans-retinoic acid and 13-cis-retinoic acid. Other suitable drugs include calciprotriene, calcitriol, calciprotiol, Vitamin D3, tocopheryl-retinoate [tocopherol ester of retinoic acid (trans- or cis-)], retinol, retinyl palmitate, retinyl acetate, retinyl propionate, retinal, and combinations thereof. The drug is typically present in an amount no greater than 1, alternatively no greater than 0.5, alternatively from 0.0001 to 0.5, alternatively no greater than 0.1, alternatively no greater than 0.05, parts by weight, based on 100 parts by weight of the composition. Selecting an appropriate amount of the drug depends in part on potency of the drug, and a desired level of treatment. It is to be appreciated that the composition can include a combination of two or more of the aforementioned drugs.

The salt of a dialkyl sulfosuccinate is typically dioctyl sodium sulfosuccinate (DOSS), which is also referred to in the art as docusate sodium or sodium docusate. As understood in the art, DOSS generally has a high melting about, of approximately 150 to 160°C. The salt of a dialkyl sulfosuccinate is especially useful for solubilizing the drug and for regulating viscosity of the composition, and can even serve as a penetration enhancer for the drug via partition coefficient. Specifically, in certain embodiments, the drug is completely solubilized and typically remains stable in the composition without agglomerating or crystallizing within the composition, once formed. Large crystal formation, such as crystals having average diameters of greater than 10 microns, and agglomerations of the crystals of the drug are undesirable as they tend to hinder the drug's effectiveness and can also be aesthetically displeasing both visually and tactilely to a consumer of the composition. Typically, since the salt of a dialkyl sulfosuccinate, in combination with the solubilizer (described below), solubilizes the drug, no heat or pH adjustment is needed in order to make the composition, e.g. to incorporate the drug within the composition. As understood in the art, heat and pH can cause some drugs to lose effectiveness by degradation. Many drug degradation products are toxic, which are undesirable. The salt of a dialkyl sulfosuccinate is typically present in an amount of 0.01 to 5, alternatively from 0.05 to 5, alternatively from 0.1 to 5, alternatively from 0.5 to 5, parts by weight, based on 100 parts by weight of the composition. Increasing the amount of the salt of a dialkyl sulfosuccinate generally decreases irritation imparted by the drug, if such an issue arises. Viscosity of the composition can also be altered, based on employing differing amounts of the salt of a dialkyl sulfosuccinate, among changing amounts and types of the other components of the composition.

In certain embodiments, depending in part on the amount of the drug employed in the composition, some portion of the drug will not be completely solubilized. In other words, in these embodiments, some amount of the drug will be solubilized and some amount of the drug will be in a disperse phase, typically in the form of small crystals having an average diameter of no greater than 10 microns, more typically having an average diameter from about 5 to about 10 microns. In these embodiments, crystal formation is controlled, such that agglomeration and therefore settling of the drug crystals is slowed, minimized, or nonexistent, due in part to the viscosity of the composition. Further, the composition is typically at steady-state, such that when the composition is employed by a consumer, the drug that is solubilized is absorbed into a consumer's skin, and the drug that is absorbed is replaced by drug that is then solubilized from the disperse phase. In other words, the crystals, if present, are solubilized from the disperse phase replace the pre-solubilized drug that is adsorbed and/or permeates into the consumer's skin, such that a near constant rate of drug diffusion of the drug is achieved until the drug is absorbed into the consumer's skin from the composition.

The solubilizer is useful for imparting homogeneity to the composition, drug solubilization, and based on its partition coefficient, the solubilizer can be a penetration enhancer for permeation into skin. For example, the solubilizer is useful for softening psoriasis plaques, to allow the drug to treat underlying skin cells. The solubilizer is especially useful for solubilizing the salt of a dialkyl sulfosuccinate, e.g. DOSS. The solubilizer, in combination with the salt of a dialkyl sulfosuccinate, is especially useful for dispersing, suspending and/or solubilizing the drug in the form of encapsulated drugs, vesicles, or other thermodynamically stable associative phases or structures. Further, the solubilizer provides the ability to coat a substrate, e.g. skin, and provides additional sensory benefits for a consumer.

The solubilizer is typically a liquid, more typically a liquid that has no melting point. In certain embodiments, the solubilizer is a liquid at a temperature greater than 0°C, alternatively greater than 1°C, alternatively greater than 5°C, alternatively greater than 20°C. In other words, the solubilizer is a liquid at temperatures typically encountered if the composition were to be refrigerated, and at temperatures typically encountered during use and/or storage of the composition.

In one embodiment, the solubilizer comprises a surfactant. The surfactant is typically a nonionic surfactant, although other types of surfactants may also be used. The surfactant typically has a hydrophilic-lipophilic balance (HLB) of less than 15, alternatively less than 14, alternatively less than 12, alternatively less than 10, alternatively less than 8, alternatively less than 7. Generally, decreasing the HLB balance increase effectiveness of the drug. Examples of suitable surfactants, for purposes of the present invention, include, but are not limited to, polysorbates, such as polysorbate 80 and polysorbate 85,and sorbitan esters, such as sorbitan monolaurate, sorbitan monoleate, sorbitan trioleate, and sorbitan sesquioleate.

In another embodiment, the solubilizer comprises a glycol. The glycol can comprise various glycols known in the art, and is typically selected from the group of propylene glycol, dipropylene glycol, butylene glycol, and combinations thereof. In one embodiment, the solubilizer is a silicone alkylmethyl glycol, which is typically present in an amount of 50 to 70 parts by weight, based on 100 parts by weight of the solubilizer. In this embodiment, the solubilizer further includes dodecane, which is present in the remaining parts by weight, based on 100 parts by weight of the mixture. A suitable example of such a solubilizer is Dow Corning^{®} Emulsifier 10, commercially available from Dow Coming Corporation. It is to be appreciated that the composition can include a combination of two or more of the aforementioned solubilizers, e.g. propylene glycol and sorbitan trioleate, dipropylene glycol and propylene glycol, etc. Optimum composite partition coefficients can be formulated by combining solubilizers with a wide range of partition coefficients. The solubilizer is typically present in an amount of 0.01 to 10, alternatively from 0.01 to 5, alternatively from 0.5 to 2, parts by weight, based on 100 parts by weight of the composition.

Typically, the salt of a dialkyl sulfosuccinate and the solubilizer are combined to form a blend (also referred to herein as a "first blend"), prior to adding additional components of the composition. Blending the solubilizer with the salt of a dialkyl sulfosuccinate, e.g. DOSS, is useful for solubilizing the DOSS, which is a solid, with the solubilizer, which is a liquid, to form the blend which itself is a liquid due to the solubilization of the DOSS. A detailed method of forming the composition is described further below. The liquid blend is particularly useful for solubilizing the drug, as described further below. In certain embodiments, the salt of a dialkyl sulfosuccinate and the solubilizer are present in a weight ratio of from 2:1 to 1:10 (either in the blend or the composition as a whole). Such weight ratios can be useful for altering release rates of the drug. Generally, a desired level of occlusiveness can be achieved by increasing the amount of the solubilizer relative to the salt of a dialkyl sulfosuccinate. Drugs with a wide solubility range can also be included in the composition based on altering the weight ratio. Specifically, optimizing solubility parameters of a blend of the salt of a dialkyl sulfosuccinate and the solubilizer allows various drugs to be employed. Transepidermal water loss can also be inhibited by adjusting the aforementioned weight ratios. Drug release and permeation of the skin can also be increased or decreased by increasing or decreasing the amount of a blend of the salt of a dialkyl sulfosuccinate and the solubilizer in the composition, or the amounts thereof in the blend itself.

The composition may further comprise an additive known to those of ordinary skill in the healthcare art. For example, the additive can be selected from the group of an emollient; a penetration enhancer; a fragrance; an antioxidant such as butylhydroxytoluene (BHT); UV light blockers such as titanium dioxide (TiO₂); steroids such as betamethasone; pH adjusters such as acids or bases, e.g. sodium bicarbonate or sodium citrate; and combinations thereof.

If employed, the emollient may be any emollient known in the art. The emollient is useful for softening and soothing skin of a consumer of the composition. The emollient is typically selected from the group of diisopropyl adipate, diisopropyl sebacate, triethyl citrate, isopropyl myristate, isopropyl palmitate, myristyl propionate, 2-ethylhexyl palmitate, cetyl palmitate, cetyl stearate, triglycerides, fatty acids, fatty alcohols (e.g. an oleyl alcohol), and combinations thereof. A suitable triglyceride is capryliclcapric triglyceride and a suitable fatty acid is caprylic acid. If employed, the emollient is typically present in an amount of 0.1 to 50, alternatively from 0.1 to 25, alternatively from 0.1 to 10, alternatively from 0.1 to 5, parts by weight, based on 100 parts by weight of the composition. In addition to solubilizing the drug into the mixture, it is believed that the salt of a dialkyl sulfosuccinate, e.g. DOSS, is useful for imparting compatibility between the mixture and the emollient, e.g. oleyl alcohol, if employed. For example, if one were to mix oleyl alcohol with just the mixture, the two would separate. However, with the addition of a suitable amount of the salt of a dialkyl sulfosuccinate, separation between the mixture and the emollient generally does not occur. Such an amount of the salt of a dialkyl sulfosuccinate depends on amounts of each of the components, and can be determined via routine experimentation.

If employed, the penetration enhancer may be any penetration enhancer known in the art. The penetration enhancer is useful for facilitating penetration of the drug into the skin of a consumer of the composition. The penetration enhancer is typically selected from the group of oleic acid, linoleic acid, glycerin monolaurate, azone (1-dodecylazacycloheptan-2-one), dodecyl 2-(N,N-dimethylamino)propionate, dodecyl 2-(N,N-dimethylamino)propionate-HCl, propylene glycol monolaurate, propylene glycol, dipropylene glycol, butylene glycol, and combinations thereof. Further examples of suitable penetration enhancers (or percutanious enhancers, are commercially available from NexMed^{®} Inc. of East Windsor, NJ, under the trade name NexACT^{®}, such as NexACT^{®} 88 Acid and NexACT^{®} 88 Base. Depending on specific components employed, the penetration enhancer can be the same as the solubilizer, such as a glycol, e.g. propylene glycol, oleic acid, and linoleic acid. If employed, the penetration enhancer is typically present in an amount of 0.01 to 5, alternatively from 0.01 to 2.5, alternatively from 0.5 to 1, parts by weight, based on 100 parts by weight of the composition.

The composition may be prepared by combining the silicone, the solvent, the drug, the salt of a dialkyl sulfosuccinate, and the solubilizer. In one embodiment, the salt of a dialkyl sulfosuccinate is solubilized in the solubilizer to form a first blend. For example, the solubilizer (liquid) can dissolve DOSS (solid). The first blend is generally a eutectic blend, as understood in the art. The drug is then mixed with the first blend to form a second blend, where the drug is solubilized by the first blend. By being eutectic, use of heat is not required to form the composition, since the salt of a dialkyl sulfosuccinate is solubilized by the solubilizer, rather than being melted with heat to reach a liquid state. The mixture of the silicone and the solvent, e.g. cyclomethicone and dimethicone crosspolymer, is then added and mixed with the second blend as a viscosity builder and matrix to make the composition; however, an opposite order of addition can also be followed. Optionally, the additive such as the emollient and/or the penetration enhancer is added to the composition. If employed. the additive can be added at various stages when making the composition, such as added with the first and/or second blends, or to composition after forming. The composition can be made in a vessel, e.g. a stainless steel vessel, using various mixing and dispersion blades, such as a propeller mixer or dental mixer. If the drug is susceptible to oxidation, nitrogen purging/sparging can be used with the vessel. Typically, a pH change is not necessary to prepare the composition; however, the pH adjuster can be employed to compensate for trace amounts of acids or bases present in the composition. In addition, application of heat is optional, depending on the salt of a dialkyl sulfosuccinate employed, and if heat is applied to the salt of a dialkyl sulfosuccinate, heat is not necessary for the composition as a whole.

If the silicone adhesive, e.g. PSA, is employed, the salt of a dialkyl sulfosuccinate is solubilized in the solubilizer to form a first blend. The drug is then mixed with first blend to form a second blend. The second blend is then mixed with the silicone adhesive (including the solvent) to form the composition. The composition can then be coated on a release liner and the solvent is allowed to volatize off via heat or ambient conditions, which results in a film, solid matrix, or patch, which can be applied to skin to provide a systemic or local therapeutic effect via the drug.

As alluded to above, the composition prepared herein can be applied to a substrate to deliver the drug. Upon application of the composition, a film is typically formed on the substrate. The film contains the drug, which is delivered from or through the film to the substrate. If the substrate is skin, the composition is applied to the skin to deliver the drug to the skin. The composition may be applied in various ways, such as by rubbing or coating the composition directly onto the skin, e.g. onto a psoriasis plaque. Alternatively, as described above, the composition may be applied on a transdermal patch prior to application of the transdermal patch to the skin.

The following examples, illustrating the compositions of the present invention, are intended to illustrate the present invention.

### EXAMPLES

Examples 1 through 50 of the composition of the present invention are prepared. The compositions are made by combining the mixture of the silicone and the solvent, the drug, the salt of a dialkyl sulfosuccinate, the solubilizer, and optionally, one of more of the additives, e.g. the emollient and the penetration enhancer, using standard blending methods. Generally, the salt of a dialkyl sulfosuccinate is solubilized in the solubilizer to form a first blend. The drug is then solubilized in the first blend to form a second blend. The second blend is then mixed with the mixture to form the composition. Depending on the solvent employed, the solvent may or may not volatilize from the composition. Commonly used stainless steel vessels are used for manufacturing the compositions. A closed vessel or tank with nitrogen purging/sparging may be necessary if the drug is susceptible to oxidation, or photo-oxidation. Various mixing means may be employed, such as propeller mixers or dental mixers. The compositions are allowed to sit undisturbed to visually determine if any settling or separation occurs. The compositions are also inspected under microscope, specifically under a 100X magnification, to determine if any agglomerations or crystals form within the compositions, especially with regard to the drug. The amount and type of each component used to form the composition is indicated in Tables 1 through 5 below with all values in parts by weight based on 100 parts by weight of the composition unless otherwise indicated.

**TABLE 1**

| **Example** | **1** | **2** | **3** | **4** | **5** | **6** | **7** | **8** | **9** | **10** |
|---|---|---|---|---|---|---|---|---|---|---|
| Mixture 1 | qs | qs | qs | qs | qs | qs | qs | qs | qs | qs |
| | | | | | | | | | | |
| Drug 1 | 0.1 | 0.1 | 0.1 | 0.1 | 0.1 | 0.1 | 0.1 | 0.1 | 0.1 | 0.1 |
| | | | | | | | | | | |
| Salt of a Dialkyl Sulfosuccinate | 1.5 | 3.0 | 3.0 | 1.5 | 1.5 | 1.5 | 3.0 | 3.0 | 1.5 | 1.5 |
| | | | | | | | | | | |
| Solubilizer 1 | 5.0 | - | - | - | - | 5.0 | - | - | - | - |
| Solubilizer 2 | - | 3.0 | - | - | - | - | 3.0 | - | - | - |
| Solubilizer 3 | - | - | 3.0 | - | - | - | - | 3.0 | - | - |
| Solubilizer 4 | - | - | - | 5.0 | - | - | - | - | 5.0 | - |
| Solubilizer 5 | - | - | - | - | 5.0 | - | - | - | - | 5.0 |
| | | | | | | | | | | |
| Emollient | - | - | - | - | - | 4.0 | 4.0 | 4.0 | 4.0 | 4.0 |

Mixture I is a high molecular weight silicone elastomer in decamethylcyclopentasiloxane (D5) in the form of a gel, commercially available from Dow Coming Corporation of Midland, MI under the trade name of Dow Corning^{®} ST-Elastomer 10. The high molecular weight silicone elastomer is present in an amount of 12 to 13 parts by weight, and the D5 is present in an amount of 87 to 88 parts by weight, each based on 100 parts by weight of the mixture.

Salt of a Dialkyl Sulfosuccinate is dioctyl sodium sulfosuccinate (DOSS), commercially available from Spectrum Chemical Mfg. Corp. of Gardena, CA.

Drug 1 is cis-retinoic acid.

Solubilizer 1 is sorbitan monolaurate.

Solubilizer 2 is dipropylene glycol.

Solubilizer 3 is propylene glycol.

Solubilizer 4 is sorbitan monoleate.

Solubilizer 5 is Polysorbate 85.

Emollient is diisopropyl sebacate, commercially available from Croda Inc. of Edison, NJ.

**TABLE 2**

| **Example** | **11** | **12** | **13** | **14** | **15** | **16** | **17** | **18** | **19** | **20** |
|---|---|---|---|---|---|---|---|---|---|---|
| Mixture I | qs | qs | qs | qs | qs | qs | qs | qs | qs | qs |
| | | | | | | | | | | |
| Drug 1 | - | - | - | - | - | - | - | - | - | - |
| Drug 2 | 0.1 | 0.1 | 0.1 | 0.1 | 0.1 | 0.1 | - | 0.1 | 0.1 | 0.025 |
| Drug 3 | - | - | - | - | - | - | 0.3 | - | - | - |
| | | | | | | | | | | |
| Salt of a Dialkyl Sulfosuccinate | 1.5 | 3.0 | 3.0 | 1.5 | 1.5 | 0.1 | 2.0 | 2.0 | 1.0 | 0.5 |
| | | | | | | | | | | |
| Solubilizer 1 | 5.0 | - | - | - | - | - | - | - | - | - |
| Solubilizer 2 | - | 3.0 | - | - | - | - | - | - | - | - |
| Solubilizer 3 | - | - | 3.0 | - | - | - | 5.0 | - | - | - |
| Solubilizer 4 | - | - | - | 5.0 | - | - | - | - | - | - |
| Solubilizer 5 | - | - | - | - | 5.0 | 7.0 | - | - | 4 | 2 |
| Solubilizer 6 | - | - | - | - | - | - | - | 8 | - | - |
| | | | | | | | | | | |
| Emollient | - | - | - | - | - | - | - | - | - | - |

Drug 2 is trans-retinoic acid.

Drug 3 is adapalene.

Solubilizer 6 is sorbitan trioleate.

**TABLE 3**

| **Example** | **21** | **22** | **23** | **24** | **25** | **26** | **27** | **28** | **29** | **30** | **31** |
|---|---|---|---|---|---|---|---|---|---|---|---|
| Mixture 1 | qs | qs | qs | qs | qs | qs | qs | qs | qs | qs | qs |
| | | | | | | | | | | | |
| Drug 1 | - | - | - | - | - | - | - | - | - | - | - |
| Drug 2 | - | - | 0.1 | 0.1 | 0.1 | 0.1 | 0.1 | 0.1 | 0.1 | 0.1 | 0.1 |
| Drug 3 | - | - | - | - | - | - | - | - | - | - | - |
| Drug 4 | 0.1 | 0.1 | - | - | - | - | - | - | - | - | - |
| | | | | | | | | | | | |
| Salt of a Dialkyl Sulfosuccinate | 2.0 | 2.0 | 2.5 | 2.0 | 5.0 | 4.0 | 2.0 | 4.0 | 2.0 | 4.0 | 4.0 |
| | | | | | | | | | | | |
| Solubilizer 1 | - | - | - | - | - | - | - | - | - | - | - |
| Solubilizer 2 | 6.0 | - | - | - | - | 4.0 | 6.0 | - | - | - | - |
| Solubilizer 3 | - | 6.0 | - | - | - | - | - | 4.0 | - | - | - |
| Solubilizer 4 | - | - | - | - | - | - | - | - | - | - | - |
| Solubilizer 5 | - | - | - | - | - | - | - | - | - | - | - |
| Solubilizer 6 | - | - | 5.0 | 8.0 | 5.0 | - | - | - | - | - | - |
| Solubilizer 7 | - | - | - | - | - | - | - | - | 8.0 | 4.0 | 6.0 |
| | | | | | | | | | | | |
| Emollient | - | - | - | - | - | - | - | - | - | - | - |

Drug 4 is tazarotene.

Solubilizer 7 is sorbitan sesquioleate.

**TABLE 4**

| **Example** | **32** | **33** | **34** | **35** | **36** | **37** | **38** | **39** |
|---|---|---|---|---|---|---|---|---|
| Mixture 1 | qs | qs | qs | qs | qs | qs | qs | qs |
| | | | | | | | | |
| Drug 1 | - | - | - | - | - | - | - | - |
| Drug 2 | - | - | - | - | - | - | - | - |
| Drug 3 | - | - | - | - | - | - | - | - |
| Drug 4 | - | - | - | - | - | - | - | - |
| Drug 5 | 0.005 | 0.005 | 0.005 | 0.005 | 0.005 | 0.005 | 0.005 | 0.005 |
| | | | | | | | | |
| Salt of a Dialkyl Sulfosuccinate | 1.0 | 1.0 | 2.0 | 2.0 | 2.0 | 2.0 | 2.0 | 2.0 |
| | | | | | | | | |
| Solubilizer 1 | - | - | - | - | - | - | - | - |
| Solubilizer 2 | - | - | 2.0 | 4.0 | 3.0 | 1.0 | - | - |
| Solubilizer 3 | - | - | - | - | - | - | - | 4.0 |
| Solubilizer 4 | - | - | - | - | - | - | - | - |
| Solubilizer 5 | - | - | - | - | - | - | - | - |
| Solubilizer 6 | 2.0 | - | - | - | - | - | 2.0 | - |
| Solubilizer 7 | - | 4.0 | - | - | - | - | - | - |
| | | | | | | | | |
| Emollient | - | - | - | - | - | - | - | - |

Drug 5 is calciprotriene.

**TABLE 5**

| **Example** | **40** | **41** | **42** | **43** | **44** | **45** | **46** | **47** | **48** | **49** | **50** |
|---|---|---|---|---|---|---|---|---|---|---|---|
| Mixture 1 | qs | qs | qs | qs | qs | qs | qs | qs | qs | qs | qs |
| | | | | | | | | | | | |
| Drug 1 | - | - | - | - | - | - | - | - | - | - | - |
| Drug 2 | 0.1 | 0.1 | 0.1 | 0.1 | - | 0.1 | 0.1 | 0.1 | 0.1 | 0.1 | 0.1 |
| Drug 3 | - | - | - | - | - | - | - | - | - | - | - |
| Drug 4 | - | - | - | - | - | - | - | - | - | - | - |
| Drug 5 | - | - | - | - | - | - | - | - | - | - | - |
| Drug 6 | - | - | - | - | 0.1 | - | - | - | - | - | - |
| | | | | | | | | | | | |
| Salt of a Dialkyl Sulfosuccinate | 2.0 | 2.0 | 3.0 | 2.0 | 2.0 | 1.0 | 2.0 | 1.0 | 2.0 | 2.0 | 1.5 |
| | | | | | | | | | | | |
| Solubilizer 1 | - | - | - | - | - | - | - | - | - | - | - |
| Solubilizer 2 | - | 2.0 | 3.0 | 4.0 | 4.0 | - | - | - | - | 4.0 | - |
| Solubilizer 3 | - | - | - | - | - | - | - | - | - | - | - |
| Solubilizer 4 | - | - | - | - | - | - | - | - | - | - | - |
| Solubilizer 5 | - | - | - | - | - | - | - | - | - | - | - |
| Solubilizer 6 | - | - | - | - | - | - | - | - | - | - | - |
| Solubilizer 7 | 5.0 | 2.5 | - | - | - | - | - | - | - | - | - |
| Solubilizer 8 | - | - | - | - | - | 6.0 | 8.0 | 7.0 | 6.0 | - | - |
| Solubilizer 9 | - | - | - | - | - | - | - | - | - | - | 6.0 |
| | | | | | | | | | | | |
| Emollient | - | - | - | - | - | - | - | - | - | - | - |
| | | | | | | | | | | | |
| Pentration Enhancer 1 | - | - | - | 0.5 | - | - | - | - | - | - | - |
| Pentration Enhancer 2 | - | - | - | - | - | - | - | 1.0 | - | - | - |
| Pentration Enhancer 3 | - | - | - | - | - | - | - | - | 0.25 | - | - |
| Pentration Enhancer 4 | - | - | - | - | - | - | - | - | - | 0.5 | - |
| | | | | | | | | | | | |
| Steroid | - | - | - | - | 1 | - | - | - | - | - | - |

Drug 6 is calciprotiol.

Solubilizer 8 is a silicone based surfactant comprising silicone alkylmethyl glycol and dodecane, commercially available from Dow Coming Corporation. The silicone alkylmethyl glycol is present in an amount of greater than 60 parts by weight, and the dodecane is present in an amount from 7 to 13 parts by weight, each based on 100 parts by weight of the silicone based surfactant.

Solubilizer 9 is butylene glycol.

Penetration Enhancer 1 is linoleic acid.

Penetration Enhancer 2 is NexACT^{®} 88 Base, commercially available from NexMed^{®}, Inc. of East Windsor, NJ.

Penetration Enhancer 3 is NexACT^{®} 88 HCl, commercially available from NexMed^{®}, Inc.

Penetration Enhancer 4 is oleic acid.

Steroid is betamethasone.

The examples above show that the drugs are soluble and compatible in the compositions of the present invention. It is believed that this is due to the synergistic solubilizing property of DOSS, especially when solubilized in the solubilizer. The synergistic solubilizing property is able to compatibilize hydrophilic drugs in lipophilic materials such as the mixtures of the silicones and the solvents, and prevents uncontrolled crystal growth over time.

The amounts of the salt of a dialkyl sulfosuccinate, e.g. DOSS, and the solubilizer, can be adjusted to maintain near saturation of the drug for optimum release of drugs.

The compositions do not have a deleterious medium that can cause instability of the drug, such as water, and no pH or heat are required for preparing the compositions of the present invention. The compositions described herein also enhance chemical stability of the drugs via increased viscosities, such as when in film form, such as with the use of a PSA, or during storage, such as with the use of a silicone elastomer based mixture, e.g. a cyclomethicone and dimethicone crosspolymer. Since the drug is solubilized (or encapsulated) via the salt of a dialkyl sulfosuccinate, e.g. DOSS, is generally in a high viscosity medium, and is substantially free of water, the compositions of the present invention have enhanced chemical stability 5 with regard to the drug.

## Claims

1. A composition comprising:
a mixture of a silicone and a solvent;
a drug selected from the group of retinoids, retinyls, vitamin A esters, vitamin D analogues, and combinations thereof;
a salt of a dialkyl sulfosuccinate for solubilizing said drug; and
a solubilizer for solubilizing said salt of a dialkyl sulfosuccinate.

2. A composition as set forth in claim 1 wherein said solubilizer comprises a surfactant.

3. A composition as set forth in claim 2 wherein said surfactant is a nonionic surfactant.

4. A composition as set forth in claims 2 or 3 wherein said surfactant has a hydrophilic-lipophilic balance (HLB) of less than 15.

5. A composition as set forth in claims 1 or 2 wherein said solubilizer comprises a glycol.

6. A composition as set forth in any preceding claim substantially free of an alcohol having the formula CₙH₂ₙ₊₁OH wherein n is from 1 to 5.

7. A composition as set forth in any preceding claim wherein said silicone is a silicone elastomer.

8. A composition as set forth in claim 7 wherein said solvent comprises a polysiloxane for swelling said silicone elastomer, and wherein said polysiloxane and said silicone elastomer are present in a weight ratio of 5:1 1 to 9:1.

9. A composition as set forth in any preceding claim wherein said salt of a dialkyl sulfosuccinate comprises dioctyl sodium sulfosuccinate (DOSS).

10. A composition as set forth in any one of claims 1 to 6 or 9 wherein said silicone is a silicone adhesive comprising the reaction product of:
a polydialkylsiloxane end blocked with silanols, and
a silicate resin.

11. A composition as set forth in claim 10 wherein said solvent is selected from the group of alkanes, arenes, esters, and combinations thereof.

12. A composition as set forth in any preceding claim wherein said drug is selected from the group of trans-retinoic acid, cis-retinoic acid, adapalene, tazarotene, calciprotriene, calcitriol, calciprotiol, vitamin D₃, and combinations thereof.

13. A composition as set forth in any preceding claim further comprising an additive selected from the group of an emollient, a penetration enhancer, a fragrance, an antioxidant, UV light blockers, steroids, pH adjusters, and combinations thereof.

14. A composition as set forth in any preceding claim wherein said salt of a dialkyl sulfosuccinate and said solubilizer are present in a weight ratio of from 2:1 to 1:10.

15. A method of delivering a drug to a substrate, said method comprising the step of applying the composition of any preceding claim to the substrate.

## Patentansprüche

1. Zusammensetzung, die umfasst:
eine Mischung eines Silikons und eines Lösungsmittels;
einen Wirkstoff, ausgewählt aus der Gruppe aus Retinoiden, Retinylen, Vitamin-A-Estern, Vitamin-D-Analogen und Kombinationen davon;
ein Salz eines Dialkylsulfosuccinats zur Solubilisierung des genannten Wirkstoffs; und
einen Lösungsvermittler zur Solubilisierung des genannten Salzes eines Dialkylsulfosuccinats.

2. Zusammensetzung wie in Anspruch 1 dargelegt, wobei der genannte Lösungsvermittler ein Surfactant umfasst.

3. Zusammensetzung wie in Anspruch 2 dargelegt, wobei das genannte Surfactant ein nichtionisches Surfactant ist.

4. Zusammensetzung wie in den Ansprüchen 2 oder 3 dargelegt, wobei das genannte Surfactant ein Hydrophile-Lipophile-Gleichgewicht (HLB) von weniger als 15 hat.

5. Zusammensetzung wie in den Ansprüchen 1 oder 2 dargelegt, wobei der genannte Lösungsvermittler ein Glykol umfasst.

6. Zusammensetzung wie in einem vorangehenden Anspruch dargelegt, die im Wesentlichen frei ist von einem Alkohol mit der Formel CₙH₂ₙ₊₁OH, wobei n von 1 bis 5 reicht.

7. Zusammensetzung wie in einem vorangehenden Anspruch dargelegt, wobei das genannte Silikon ein Silikonelastomer ist.

8. Zusammensetzung wie in Anspruch 7 dargelegt, wobei das genannte Lösungsmittel ein Polysiloxan zum Quellen des genannten Silikonelastomers umfasst, und wobei das genannte Polysiloxan und das genannte Silikonelastomer in einem Gewichtsverhältnis von 5:1 bis 9:1 vorliegen.

9. Zusammensetzung wie in einem vorangehenden Anspruch dargelegt, wobei das genannte Salz eines Dialkylsulfosuccinats Dioctylnatriumsulfosuccinat (DOSS) umfasst.

10. Zusammensetzung wie in einem der Ansprüche 1 bis 6 oder 9 dargelegt, wobei das genannte Silikon ein Silikonkleber ist, der das Reaktionsprodukt aus:
einem Polydialkylsiloxan, dessen Enden mit Silanolen geblockt sind, und
einem Silikatharz
umfasst.

11. Zusammensetzung wie in Anspruch 10 dargelegt, wobei das genannte Lösungsmittel ausgewählt ist aus der Gruppe aus Alkanen, Arenen, Estern und Kombinationen davon.

12. Zusammensetzung wie in einem vorangehenden Anspruch dargelegt, wobei der genannte Wirkstoff ausgewählt ist aus der Gruppe aus trans-Retinsäure, cis-Retinsäure, Adapalen, Tazaroten, Calciprotrien, Calcitriol, Calciprotiol, Vitamin D₃ und Kombinationen davon.

13. Zusammensetzung wie in einem vorangehenden Anspruch dargelegt, die weiterhin ein Additiv, ausgewählt aus der Gruppe aus einem Emolliens, einem Penetrationsverstärker, einem Duftstoff, einem Antioxidationsmittel, UV-Licht-Blockern, Steroiden, Mitteln zur Einstellung des pHs und Kombinationen davon, umfasst.

14. Zusammensetzung wie in einem vorangehenden Anspruch dargelegt, wobei das genannte Salz eines Dialkylsulfosuccinats und der genannte Lösungsvermittler in einem Gewichtsverhältnis von 2:1 bis 1:10 vorliegen.

15. Verfahren zum Abgeben eines Wirkstoffs an ein Substrat, wobei das genannte Verfahren den Schritt des Auftragens der Zusammensetzung gemäß einem vorangehenden Anspruch an das Substrat umfasst.

## Revendications

1. Composition comprenant :
un mélange d'une silicone et d'un solvant ;
un médicament sélectionné dans le groupe des rétinoïdes, des rétinyles, des esters de la vitamine A, des analogues de la vitamine D, et de leurs combinaisons ;
un sel d'un sulfosuccinate de dialkyle pour solubiliser ledit médicament ; et
un agent de solubilisation pour solubiliser ledit sel d'un sulfosuccinate de dialkyle.

2. Composition selon la revendication 1, dans laquelle ledit agent de solubilisation comprend un surfactant.

3. Composition selon la revendication 2, dans laquelle ledit surfactant est un surfactant non ionique.

4. Composition selon la revendication 2 ou 3, dans laquelle ledit surfactant a un équilibre hydrophile-lipophile (HLB) inférieur à 15.

5. Composition selon la revendication 1 ou 2, dans laquelle ledit agent de solubilisation comprend un glycol.

6. Composition selon l'une quelconque des revendications précédentes, substantiellement dépourvue d'un alcool de formule CₙH₂ₙ₊₁OH, dans laquelle n vaut de 1 à 5.

7. Composition selon l'une quelconque des revendications précédentes, dans laquelle ladite silicone est un élastomère de silicone.

8. Composition selon la revendication 7, dans laquelle ledit solvant comprend un polysiloxane destiné à faire gonfler ledit élastomère de silicone, et dans laquelle ledit polysiloxane et ledit élastomère de silicone sont présents en un rapport en poids de 5/1 à 9/1.

9. Composition selon l'une quelconque des revendications précédentes, dans laquelle ledit sel d'un sulfosuccinate de dialkyle comprend du dioctylsulfosuccinate de sodium (DOSS).

10. Composition selon l'une quelconque des revendications 1 à 6 ou 9, dans laquelle ladite silicone est un adhésif à base de silicone comprenant un produit de réaction de :
un polydialkylsiloxane à extrémités bloquées par des groupes silanol, et
une résine de silicate.

11. Composition selon la revendication 10, dans laquelle ledit solvant est sélectionné dans le groupe des alcanes, des arènes, des esters, et de leurs combinaisons.

12. Composition selon l'une quelconque des revendications précédentes, dans laquelle ledit médicament est sélectionné dans le groupe de l'acide transrétinoïque, de l'acide cis-rétinoïque, de l'adapalène, du tazarotène, du calciprotriène, du calcitriol, du calciprotiol, de la vitamine D₃, et de leurs combinaisons.

13. Composition selon l'une quelconque des revendications précédentes, comprenant en outre un additif qui est sélectionné dans le groupe d'un émollient, d'un agent améliorant la pénétration, d'un parfum, d'un antioxydant, des agents bloquant les UV, des stéroïdes, des agents d'ajustement du pH, et de leurs combinaisons.

14. Composition selon l'une quelconque des revendications précédentes, dans laquelle ledit sel de sulfosuccinate de dialkyle et ledit agent de solubilisation sont présents en un rapport en poids de 2/1 à 1/10.

15. Procédé d'administration d'un médicament à un substrat, ledit procédé comprenant l'étape consistant à appliquer la composition selon l'une quelconque des revendications précédentes au substrat.
